# FASCICULE DE BREVET EUROPEEN

(11) **EP 3 194 423 B1**
(45) Date de publication et mention de la délivrance du brevet: **09.10.2019**
(21) Numéro de dépôt: 15771175.5
(22) Date de dépôt: 28.08.2015
(51) Int. Cl.: C07K 14/11, A61K 39/145, C07C 39/17

(54) **PROCÉDÉ DE PRÉPARATION D'UN ANTIGÈNE VACCINAL, ANTIGÈNE VACCINAL OBTENU ET UTILISATIONS**
VERFAHREN ZUR HERSTELLUNG EINES IMPFSTOFFANTIGENS, SO ERHALTENES IMPFSTOFFANTIGEN UND VERWENDUNGEN
METHOD FOR PREPARING A VACCINE ANTIGEN, RESULTING VACCINE ANTIGEN AND USES

(30) Priorité: 29.08.2014 FR 1458124
(43) Date de publication de la demande: 26.07.2017
(73) Titulaire: Calixar, 69008 Lyon (FR); Université Claude Bernard Lyon 1, 69100 Villeurbanne (FR); Hospices Civils De Lyon, 69002 Lyon (FR); Inserm, 75654 Paris Cedex 13 (FR)
(72) Inventeur: ROSA-CALATRAVA, Manuel, F-69003 Lyon (FR); TRAVERSIER, Aurélien, F-69200 Vénissieux (FR); DESUZINGES-MANDON, Élodie, F-69230 Saint-Genis-Laval (FR); DEJEAN, Emmanuel, F-69360 Solaize (FR)
(74) Mandataire: Novagraaf Technologies
(86) Numéro de dépôt international: PCT/FR2015/052285
(87) Numéro de publication internationale: WO 2016/030635

(56) Documents cités:
- WO-A1-2009/029695
- WO-A1-2009/144419
- WO-A1-2011/051235
- CORRADA GERACI ET AL: "First Self-Adjuvant Multicomponent Potential Vaccine Candidates by Tethering of Four or Eight MUC1 Antigenic Immunodominant PDTRP Units on a Calixarene Platform: Synthesis and Biological Evaluation", BIOCONJUGATE CHEMISTRY, vol. 24, no. 10, 16 octobre 2013 (2013-10-16), pages 1710-1720, XP055196253, ISSN: 1043-1802, DOI: 10.1021/bc400242y

## Description

### Domaine technique

La présente invention se rapporte à un procédé de préparation d'un antigène vaccinal comprenant une protéine membranaire, ainsi qu'à un antigène vaccinal et un vaccin, et leurs utilisations.

La présente invention trouve une application industrielle dans le domaine médical, et notamment dans le domaine de la préparation de vaccins.

Dans la description ci-dessous, les références entre crochets (**[ ]**) renvoient à la liste des références présentée à la fin du texte.

### Etat de la technique

La vaccination consiste à immuniser une personne ou un animal contre une maladie infectieuse, généralement en lui administrant un vaccin. Les vaccins, qui stimulent le système immunitaire, prémunissent la personne ou l'animal d'une infection ou d'une maladie.

Il est établi que la vaccination humaine permet ainsi de combattre et d'éliminer des maladies infectieuses potentiellement mortelles, et on estime qu'ainsi plus de 2 à 3 millions de décès par an sont évités. C'est l'un des investissements les plus rentables dans le domaine de la santé.

Un vaccin est une préparation antigénique permettant d'induire chez la personne ou l'animal qu'on vaccine, une réponse immunitaire spécifique d'un agent pathogène capable de le protéger contre l'infection naturelle ou d'en atténuer les conséquences.

La grippe est une infection respiratoire virale fréquente, observée partout dans le monde, évoluant par bouffées épidémiques hivernales dans les régions tempérées, due aux virus influenza. L'Organisation Mondiale de la Santé (OMS) estime qu'elles entraînent entre 3 et 5 millions de cas graves et 250 000 à 500 000 décès par an dans le monde (http://www.pasteur.fr/fr/institut-pasteur/presse/fiches-info/grippe#sthash.PwUNmJ10.dpuf). Les virus influenza responsables de pathologies chez l'homme sont les virus influenza de type A et B. Alors que les virus influenza de type B circulent sous forme de lignage, les virus influenza de type A sont classés en sous types viraux en fonction des propriétés antigéniques des deux glycoprotéines majeures de surface, l'Hémagglutinine (HA) et la Neuraminidase (NA). Les virus influenza présentent entre 300 et 800 glycoprotéines à leur surface associée à un ratio NA/HA pouvant aller de un pour dix.

Les virus circulants chez l'homme et responsables des épidémies saisonnières sont les virus A (H1N1) et A (H3N2). Le principal réservoir des virus influenza étant le réservoir animal (aviaire et porcin), des virus animaux peuvent franchir la barrière d'espèce et infecter l'homme. Des virus comme le virus aviaire A (H5N1) et le virus A (H1N1) pandémique peuvent entrainer de graves problèmes de santé publique.

Or, la vaccination est, pour l'instant, le seul moyen efficace pour protéger les populations des virus influenza. Le vaccin dit saisonnier permet d'acquérir une immunité contre les virus circulants saisonniers A comme (H1N1) et (H3N2) et les différents lignages de virus B. Il est défini chaque année à partir des souches prototypes de l'année antérieure et contient les antigènes des virus saisonniers de type A et de type B. La réponse immunitaire de l'hôte est principalement de type humorale avec expression d'anticorps neutralisants qui sont dirigés contre les protéines HA et NA des virus.

En raison d'une dérive antigénique importante de ces deux protéines, la composition vaccinale doit être ré-évaluée annuellement.

Le processus classique de production d'un vaccin repose dans un premier temps sur l'obtention par réassortiment génétique entre la souche A/PR8/34 (H1N1) et les souches virales saisonnières, de semences vaccinales sur oeuf, pour chacune des souches A prototypes annuelles déterminées par l'OMS. La plupart des fabricants de vaccin utilisent de manière usuelle ces virus réassortants dérivant principalement du virus parental A/PR/8/34. Ainsi, chaque semence vaccinale est issue d'un processus de réassortiment génétique entre la souche prototype A et le virus A/PR8/34 (H1N1) qui possède des capacités réplicatives optimales en oeuf. La production de virus de type B se fait directement sur oeuf sans réassortiment avec PR8.

La particule virale contient huit segments de gènes distincts, constitués d'une chaîne unique d'ARN liée à des nucléoprotéines et associés à un complexe polymérasique, chacun des gènes codant une à trois protéines déterminées du virus : HA, NA, M1, M2, NP, NS1, NEP, PB1, PB1F2, PB1N40, PB2 et PA. Par conséquent, un long processus de sélection permet l'obtention du réassortant vaccinal, comportant au minimum les segments de gènes codant pour HA et NA de la souche prototype sur le fond génétique du virus PR8 (composition « 6+2 »). Les 2 réassortants vaccinaux, issus du réassortiment génétique entre les 2 souches prototypes et la souche parentale A/PR/8/34, sont ensuite amplifiés sur oeuf. Les antigènes HA et NA sont extraits à partir des productions allantoïques, associés ou non avec des adjuvants afin de produire les doses vaccinales.

Ce processus industriel pour la fabrication des doses vaccinales est long (4 à 6 mois). Or, dans le cas de production de vaccins prépandémiques, notamment contre la grippe humaine, il a été évalué par l'OMS qu'environ 1 milliard de personnes devraient être vaccinées si un plan de lutte contre une pandémie de grippe devait être mis en oeuvre. Or, un tel plan de lutte dépendra, en partie, de l'efficacité de la production massive et rapide de vaccins.

Pour faire face à la demande croissante de vaccins contre les couches circulantes saisonnières, mais également à la demande - difficilement prévisible - de vaccins contre une (ou plusieurs) souches émergentes potentiellement pandémiques, la disponibilité des oeufs peut s'avérer être un facteur limitant, d'autant plus que des risques de pandémies aviaires dans les élevages persistent. Il existe toutefois une difficulté permanente à produire avec un bon rendement des souches B en oeufs, ainsi que certains réssortants de type A qui ne sont pas 6+2 mais plutôt 5+3 ou 4+4.

Ainsi, afin de réduire la durée entre la sélection des souches prototypes circulantes et la fabrication des doses vaccinales, des stratégies alternatives se sont développées. Les virus influenza peuvent être également produits en systèmes cellulaires (Barrett PN, Portsmouth D, Ehrlich HJ. Developing cell culture-derived pandemic vaccines. Curr Opin Mol Ther. 2010 Feb;12(1):21-30 **([1]);** Le Ru A, Jacob D, Transfiguracion J, Ansorge S, Henry O, Kamen AA. Scalable production of influenza virus in HEK-293 cells for efficient vaccine manufacturing. Vaccine. 2010 May 7;28(21):3661-71 **([2])).** La production des souches vaccinales ainsi que leurs amplifications peuvent être réalisées sur cellules en utilisant des bioréacteurs industriels. En effet, l'utilisation de lignée cellulaire pour l'amplification des souches vaccinales permet entre autre de ne plus être dépendant du système « oeuf » (quantité d'oeuf et rendement de production insuffisants pour la gestion d'une pandémie), réduit les modifications des antigènes de surface régulièrement observées dans la production allantoïque et n'entraine pas d'allergies. Cette stratégie permet de répondre plus facilement à la demande de vaccin lors de l'émergence d'un virus influenza pandémique (par exemple A H1N1).

Néanmoins ces systèmes, et notamment les lignées cellulaires enregistrées (MDCK, Vero, PERC6, EB66, etc.) ne permettent pas une réplication optimale des virus influenza, ce qui constitue un facteur limitant majeur en termes de production de semences vaccinales. Les rendements de production en systèmes cellulaires sont actuellement encore inférieurs à ceux obtenus en système allantoïque. Ainsi, à l'heure actuelle, peu d'industriels ont choisis ce nouveau mode de production car le processus industriel est loin d'être aussi performant que les oeufs pour l'instant.

Afin de réduire au minimum le temps de production des doses vaccinales et avec l'objectif d'avoir un niveau de rendement équivalent, en terme de nombre de dose, l'obtention des semences vaccinales peut être réalisée par l'utilisation des techniques de génétique inverse, permettant d'obtenir plus rapidement le réassortant vaccinal de composition "6+2", éliminant ainsi toutes les étapes de sélection. La production de virus recombinés par génétique inverse présente l'alternative la plus réaliste pour répondre efficacement à la demande de vaccins. La production de virus recombinés par génétique inverse donne la possibilité, dans un deuxième temps, de produire un virus PR8 « optimisé » permettant, lorsqu'il sera utilisé par un processus de réassortiment génétique avec des souches prototypes, de produire des réassortants vaccinaux présentant des caractéristiques virales optimisées pour la production de dose vaccinale en oeuf ou en cellule. Ce type de méthode peut également être appliquée pour la production en cellule.

Une autre stratégie pour l'optimisation de la production de vaccin en systèmes allantoïque et cellulaire, vise à augmenter le rendement de la production virale sur la base de modifications génétiques des semences vaccinales et/ou à l'aide de petites molécules chimiques d'intérêt ciblant la cellule hôte. De tels procédés sont en cours d'évaluation (FR 10/55716 **([3]),** WO 2012007380 **([4]),** FR 10/59132 **([5]),** WO 2012059696 **([6])).**

Une autre stratégie consiste à améliorer l'efficacité des antigènes vaccinaux, par l'addition d'adjuvants, ce qui permet de produire davantage de doses de vaccin pour un même nombre limité d'oeufs et une même quantité de glycoprotéines produites (Ellebedy AH, Webby RJ. Influenza vaccines. Vaccine. 2009 Nov 5;27 Suppl 4:D65-8 **([7])).** Ce procédé est davantage préconisé pour les personnes au système immunitaire déficient. Par ailleurs, l'opinion publique est plutôt réfractaire à l'utilisation des adjuvants.

Enfin, une stratégie consiste à optimiser l'étape d'extraction des antigènes vaccinaux (« Split ») en aval du procédé de production: l'objectif étant d'extraire mieux et davantage les antigènes vaccinaux en préservant plus efficacement leur structure conformationnelle et donc leur propriété antigénique dont dépend directement le pouvoir protecteur du vaccin.

Plus précisément, la production virale obtenue à partir d'oeufs embryonnés de poule infectés (récolte du liquide allantoïque) ou de culture de lignées cellulaires infectées en bioréacteur (récolte du milieu de culture) est purifiée sur gradient de sucrose puis inactivée par traitement chimique (préférentiellement à la formaline, pour l'obtention réglementaire d'une chute du titre viral de 15 logs). Le procédé met en jeu ensuite l'utilisation d'un détergent qui va déstructurer/fragmenter (« disrupting/fragmenting ») les virus (« split virions ») et une purification des antigènes vaccinaux par diafiltration.

Le split, ou virus fragmenté, obtenu, est associé à une solubilisation partielle ou totale des protéines virales, altérant ainsi l'intégrité et la fonctionnalité (infectieuses) des virus. Le Splitting constitue également une méthode d'inactivation complémentaire à celle chimique ; on parle alors d'inactivation orthogonale.

Le split peut être obtenu par le traitement des virus purifiés par différents détergents, notamment par des surfactants non-ioniques et ioniques (e.g. cationique) tels que: alkylglycosides, alkylthioglycosides, acyl sugars, sulphobétaïnes, bétaïnes, polyoxyethylenealkylethers, N,N-dialkyl-glucamides, hecameg, alkylphénoxy-polyéthoxyéthanols, composés ammonium quaternaires, sarcosyl, CTAB (cetyl trimethyl ammonium bromides), tri-N-butyl phosphate, Cetavlon, sels de myristyltrimethylammonium, lipofectine, lipofectamine, DOT-MA (bromure Dodécyltrimethylanmonium), polyoxyéthanols octyl- ou nonylphenoxy (e.g. Triton X-100 ou Triton NI 01), esters de polyoxyethylene sorbitan (surfactants Tween), éthers polyoxyethylene, éthyl éther, polysorbate 80, deoxycholate, Tergitol® NP9, etc.

A ce titre, de nombreuses méthodes de splitting (fragmentation) des virus influenza sont connues. Le document WO02/28422 **([8])** décrit par exemple une méthode de splitting au moyen d'un agent de splitting choisi parmi laureth 9, NaDOC, Sarcosyl group, Tween 80TM, and Triton X100. Le document WO02/067983 **([9])** décrit quant à lui une méthode de splitting au moyen du deoxycholate de sodium.

Les tensioactifs (ou détergents) sont des molécules amphiphiles composées de domaines polaires et apolaires bien distincts présentant une solubilité marquée dans l'eau. Au-delà d'une concentration précise appelée Concentration Micellaire Critique (CMC), le phénomène de micellisation est dirigé par l'effet hydrophobe (M. Rosen, « Surfactants and Interfacial Phenomena », 3rd Ed., Hoboken: John Wiley & Sons, Inc., 2004 **([10])).**

La nature de la queue hydrophobe, sa longueur, son degré d'insaturation et de ramification, la présence ou non de noyaux aromatiques et le nombre de chaînes affectent les propriétés chimiques des monomères de tensioactif et leur auto-assemblage en solution aqueuse (CMC, nombre d'agrégation, taille et nature de la forme géométrique des agrégats). La nature du groupement hydrophile (neutre ou chargé, petit ou volumineux) impacte fortement sur les propriétés du tensioactif et son application biologique.

Les tensioactifs sont classés en trois grandes familles selon la nature de leur partie hydrophile (A.M. Schwartz, J.W. Perry, J. Berch, « Surface Active Agents and Detergents », vol II, R. Krieger Pub. Co., New York, 1977 **([11]))** : les tensioactifs ioniques (cationiques ou anioniques), les tensioactifs zwitterioniques (ou amphotères) et les tensioactifs neutres. Ils sont souvent référencés comme étant soit doux (« soft ») ou dur (« harsh »). On peut globalement faire un ordre de classement des détergents selon leur classe des plus durs aux plus doux dans ce sens: ionique>amphotère>neutre. Les tensioactifs ioniques, comme le CTAB, sont généralement dénaturants. Ils perturbent les interactions coulombiennes intramoléculaires des protéines et ainsi désorganisent leur conformation tridimensionnelle.

Les tensioactifs zwitterioniques (sulfobétaines, bétaines) contiennent à la fois une charge positive et négative dans leur partie hydrophile et sont électriquement neutres.

Les tensioactifs neutres, incluant notamment les alkyl glucosides et les polyoxyethylenealkyléthers sont caractérisés par des têtes hydrophiles non chargées. Ce sont des tensioactifs doux et non dénaturants perturbants toutefois les interactions protéine-lipide et lipide-lipide, et sont sans effet sur les interactions coulombiennes intramoléculaires des protéines.

Les détergents comportant une partie hydrophile de type polyéthylène glycols (Triton X100, Brij®, Tween,...) sont couramment utilisés en biochimie. Ils ont le désavantage d'être considérés comme des molécules étant chimiquement hétérogènes avec un indice de polymérisation n variable sur leurs groupements PEGs (polyéthylène glycol). Ces lots de détergents du commerce ne sont donc pas sous la forme d'une espèce chimiquement bien définie en solution, ce qui peut entraîner une variation de leurs propriétés physico-chimiques.

Dans le contexte actuel de l'émergence d'un virus pathogène pandémique, une infection, par exemple grippale, pourrait entrainer 1,3 à 2 millions d'hospitalisations et de 280 000 à 650 000 décès dans les seuls pays industrialisés (données OMS).

Un des enjeux économiques majeurs est donc de pouvoir réduire le coût de fabrication d'une dose vaccinale (plus de doses par production et/ou réduction du temps d'obtention de la même quantité de doses).

Dans ce contexte et d'un point de vue économique, la recherche et la mise au point de nouveaux procédés optimisés de production des semences vaccinales sont légitimes, notamment en termes (i) d'amélioration des rendements de production, (ii) de réduction de temps et/ou des coûts, (iii) de production d'antigènes davantage immunogènes.

### Description de l'invention

La présente invention a précisément pour but de répondre à ces besoins en fournissant un procédé de préparation d'un antigène vaccinal permettant d'optimiser qualitativement et quantitativement l'étape d'extraction des antigènes vaccinaux **du virus de la grippe,** dans un procédé industriel de production, notamment en système cellulaire ou allantoïque.

La Demanderesse a en effet montré, de manière surprenante et inattendue, qu'un calixarène de formule (I), et avantageusement un calixarène de formule (II) ou l'un de leurs sels pharmaceutiquement acceptables, peuvent être utilisés pour la fragmentation (« splitting ») d'un virus, notamment en remplacement des détergents utilisés classiquement pour la fragmentation des membranes, comme par exemple le Triton X100 ou le Tween 80.

Le procédé de l'invention présente notamment l'avantage de mieux préserver la conformation des glycoprotéines membranaires telles que les antigènes vaccinaux de surface. La présente invention permet de manière surprenante une meilleure préservation de la conformation des antigènes lors de l'étape de fragmentation, notamment des virus (« Split »), qu'avec les techniques connues.

Dans ce contexte, son utilisation permet de générer des antigènes vaccinaux beaucoup plus efficaces d'un point de vue immunogénique, et ainsi de réduire les quantités d'antigènes nécessaires dans les lots de vaccin pour une protection identique ou comparable.

De manière complémentaire, ces antigènes plus efficaces car mieux préservés, permettent de manière avantageuse d'être, en tout ou partie, une alternative aux adjuvants connus. Alternativement, les calixarènes peuvent être utilisés comme adjuvants et/ou excipients dans une composition vaccinale, pour améliorer les propriétés des antigènes vaccinaux.

L'invention permet notamment d'augmenter la qualité et/ou la quantité d'antigènes vaccinaux produits en comparaison à l'utilisation classique de la molécule détergente Triton X100.

L'utilisation de l'invention est adaptable avec les différents modes de production existants (oeufs, i.e. systèmes allantoïques, et cellules) mais également avec les autres méthodes complémentaires d'optimisation de production (citées précédemment), ainsi qu'avec les modes de production en développement, comme les méthodes impliquant les feuilles de tabac, les protozoaires.

Ainsi, il est décrit un procédé de préparation d'un antigène vaccinal, comprenant une étape de fragmentation d'une membrane biologique associée audit antigène vaccinal par mise en contact de ladite membrane biologique avec au moins un calixarène de formule (I): 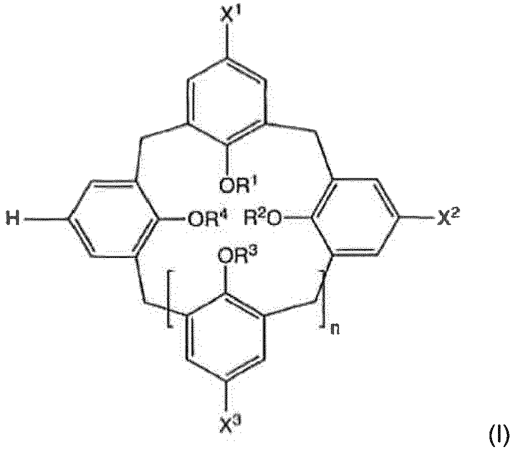 dans laquelle
- n est un nombre entier égal à 1, 3, 5 ou 6 ;
   - R¹, R², R³, R⁴ représentent, indépendamment l'un de l'autre, un atome d'hydrogène ; un groupe (C 1-18) alkyle linéaire ou ramifié éventuellement substitué par un ou plusieurs hétéroatomes choisis dans le groupe d'atomes O, S, N, P ; un groupe (C 1-18) alkyle linéaire ou ramifié éventuellement substitué par un groupe - COOR où R est un groupe (C 1-4) alkyle linéaire ou ramifié; un groupe aryle comprenant de 6 à 20 atomes de carbone;
   - X¹, X², et X³ représentent, indépendamment l'un de l'autre, un groupe -(CH₂)m-COOR' ou -SO₃ ou -(CH₂)ₘPO(OH)O, ou-(CH₂)ₘ-NR'₃, dans lequel
      - m est un nombre entier allant de 0 à 20,
      - R' représente un atome d'hydrogène ou un groupe (C 1-4) alkyle linéaire ou ramifié ;
   ou l'un de ses sels pharmaceutiquement acceptables,
dans lequel l'antigène vaccinal n'est pas un antigène du VIH (virus de l'immunodéficience humaine) ni un antigène du CMV (cytomégalovirus).

L'invention se rapporte à un procédé de préparation d'un antigène vaccinal fragmenté du virus de la grippe, comprenant une étape de fragmentation d'une membrane biologique associée audit antigène vaccinal par mise en contact de ladite membrane biologique avec au moins un calixarène de formule (II) : 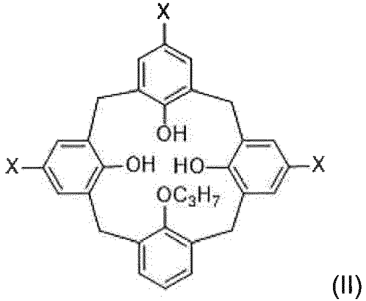 dans lequel :
- X est un groupement -(CH₂)-CO₂Y, et
- Y est un métal alcalin choisi parmi les sels de sodium, de potassium, d'ammonium, amino (organique) ou magnésium,
ou l'un de ses sels pharmaceutiquement acceptables,
dans lequel ladite étape de fragmentation est réalisée à une température allant de 4 à 25°C, et à une concentration dudit calixarène de 1%.

Avantageusement, le virus de la grippe peut être choisi parmi les virus influenza A et les virus influenza B.

Le procédé de l'invention peut comprendre en outre une étape de dialyse dudit antigène vaccinal.

Un autre objet de l'invention se rapport à un vaccin contre le virus de la grippe susceptible d'être produit par la mise en oeuvre du procédé de l'invention, comprenant un calixarène de formule (II) sous format excipient en une quantité comprise de 0,1 à 1000µg dans le poids total du vaccin.

Avantageusement, l'antigène vaccinal peut comprendre une protéine membranaire d'origine virale.

Un autre objet de l'invention se rapporte à l'utilisation d'un calixarène de formule (II) : 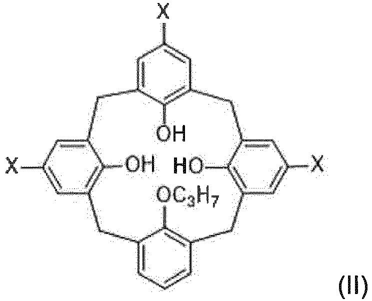 dans lequel :
- X est un groupement -(CH2)-CO2Y, et
- Y est un métal alcalin choisi parmi les sels de sodium, de potassium, d'ammonium, amino (organique) ou magnésium,
ou l'un de ses sels pharmaceutiquement acceptables,
pour la préparation d'un vaccin contre le virus de la grippe ou d'un antigène vaccinal du virus de la grippe par fragmentation d'une membrane biologique associée audit antigène vaccinal par mise en contact de ladite membrane biologique avec au moins ledit calixarène, ladite étape de fragmentation étant réalisée à une température allant de 4 à 25°C, et à une concentration dudit calixarène de 1%.

Un autre objet de l'invention se rapporte à un vaccin selon l'invention, pour son utilisation comme médicament dans le traitement ou la prévention de la grippe.

On entend par « antigène vaccinal », au sens de la présente invention, toute préparation comprenant au moins une protéine membranaire susceptible d'être exprimée par un virus. L'antigène vaccinal peut en outre comprendre un fragment d'une membrane biologique. A ce titre, la protéine membranaire peut être associée à la membrane biologique. L'antigène vaccinal s'entend également de fragments de membrane biologique hérissés d'antigènes vaccinaux, par exemple sur la face interne et/ou externe de la membrane biologique.

L'antigène vaccinal permet avantageusement une stimulation de la réponse immunitaire d'un individu, humain ou animal, à qui il est administré, contre le virus exprimant cet antigène et notamment contre la protéine membranaire. L'antigène vaccinal peut avantageusement permettre une immunisation, totale ou partielle, de l'individu contre l'antigène et notamment contre la protéine membranaire.

On entend par " protéine membranaire ", au sens de la présente invention, une protéine associée à une membrane biologique, c'est-à-dire soit ancrée, soit intégrale, non libre de diffusion dans les milieux aqueux et instable dans ces milieux. Parmi les protéines membranaires, on peut citer par exemple les protéines de membranes plasmiques et les protéines de membranes intracellulaires (comme par exemple les protéines de membranes mitochondriales, de réticulum, golgienne, lysosomales. Les protéines membranaires peuvent être des protéines polytopiques ou des protéines monotopiques. On entend par " protéines polytopiques " des protéines qui traversent une ou plusieurs fois la membrane. On entend par " protéines monotopiques " des protéines qui interagissent avec un seul coté de la membrane. La protéine membranaire peut également être une protéine intégrale ou périphérique. On entend par " protéines intégrales " des protéines monotopiques ou polytopiques qui interagissent fortement avec la membrane, notamment par des interactions hydrophobes. Ces protéines sont aussi appelées " protéines intrinsèques ". On entend par " protéines périphériques " des protéines monotopiques qui interagissent faiblement avec la membrane, c'est-à-dire soit par des liaisons électrostatiques, soit par l'intermédiaire d'autres protéines membranaires. Ces protéines sont aussi appelées " protéines extrinsèques ".

On décrit également une protéine antigénique exprimée par un agent infectieux Il est décrit un virus sauvage ou recombinant, de tout type de souche, choisi dans le groupe comprenant :
- la famille des virus orthomyxoviridae, incluant notamment les virus de la grippe, comme les les virus influenza de mammifère, et plus particulièrement les virus influenza humains, les virus influenza porcins, le virus influenza équin, le virus influenza félin, les virus influenza aviaires, comme le virus influenza du cygne ,
- la famille des virus paramyxoviridae, incluant notamment les respirovirus (sendai, virus parainfluenza bovin 3, parainfluenza 1 and 3 humain), les rubulaviruses (parainfluenza 2, 4, 4a, 4b humains, le virus humain des oreillons, parainfluenza de type 5), les avulavirus (virus de la maladie de Newcastle (NDV)), les pneumoviruses (virus respiratoires syncytiaux humain et bovin), les métapneumovirus (métapneumovirus animal et humain), les morbilivirus (virus de la rougeole, de la maladie de carré et de la peste bovine) et les henipavirus (virus hendra, nipah...etc),
- la famille des virus coronaviridae incluant notamment les coronavirus humains (notamment NL63, SARS-CoV, MERS-CoV) et animaux (coronavirus canin, porcin, bovin et de la bronchite infectieuse aviaire) ;
- la famille des virus flaviviridae incluant notamment les arbovirus (virus de l'encéphalite à tiques), les flavivirus (virus de la dengue, de la fièvre jaune, de l'encéphalite de Saint Louis, de l'encéphalite japonaise, du Nil occidental incluant le sous type Kunjin, de la Muray vallée, virus Rocio, virus Ilheus, virus de la méningo-encéphalite à tique), les hepacivirus (virus de l'hépatite C, le virus de l'hépatite A, le virus de l'hépatite B) et les pestivirus (virus de la maladie des frontières, de la diarrhée bovine, de la fièvre du cygne),
- les virus Rhabdoviridae incluant notamment les vésiculovirus (virus de la stomatite vésiculaire), les lyssavirus (virus de la rage, des chauve-souris de Lagos, australienne, européenne), les ephemerovirus (virus de la fièvre éphémère bovine), les novirhabdovirus (virus de la tête de serpent, de la septicémie hémorragique et de la nécrose hématopoïétique),
- la famille des virus Togaviridae incluant notamment les rubivirus (virus de la rubéole), les alphavirus (notamment le virus Sinbis, virus Semliki forest, virus O'nyong'nyong, virus Chikungunya, virus Mayaro, virus de Ross river, virus de l'encéphalite équine de l'Est, virus de l'encéphalite équine de l'Ouest, virus de l'encéphalite équine du Venezuela),
- la famille des virus herpesviridae incluant notamment les herpesvirus humain (HSV-1, HSV-2, le vius de la varicelle, le virus Epstein-Barr, cytomégalovirus, roséolovirus, HHV-7 et le KSHV),
- la famille des virus poxviridae incluant notamment les orthopoxvirus (comme notamment camoepox, cowpox, smallpox, vaccinia), les carpipoxvirus (incluant notamment sheepox), les avipoxvirus (incluant notamment le fowlpox), les parapoxvirus (incluant notamment le virus de la stomatite papuleuse bovine) et les leporipoxvirus (incluant notamment le virus de la myxomatose),
- la famille des virus rétroviridae incluant notamment les lentivirus (incluant notamment les virus de l'immunodéficience humaine 1 et 2, féline, simienne, le virus de l'arthrite encéphalite caprine ou de la maladie de Maedi-Visna) et les rétrovirus (incluant notamment les virus du sarcome de Roux, lymphotrophique humains 1, 2 et 3),
Un virus modifié, réassortant ou prototype produit en système cellulaire par génétique inverse est également décrit.

Une glycoprotéine virale comme la Neuraminidase ou l'Hémagglutinine, ou les protéines de membrane M1 et M2, la nucléoprotéine, la Non-Structural Protein (NS1) des virus influenza, ou par exemple de la protéine de fusion F des virus paramyxoviridae, ou toute autre protéine virale, notamment toute protéine associée à une membrane d'un virus cité précédemment est décrite. Une protéine virale modifiée, comme par exemple un mutant de la protéine de fusion F comme décrit dans WO 2010/058100 **([26])** ou WO2010/058099 **([27])** est décrite. **Un** antigène choisi parmi l'antigène de surface de membrane externe Pertactine de salmonelle et l'antigène pertactine de Bordetella pertussis est décrit.

On entend par « membrane biologique », au sens de la présente invention, tout assemblage de molécules en un double feuillet séparant une cellule de son environnement, composée d'une bicouche de lipides amphiphiles, notamment des phospholipides, chaque lipide membranaire étant constitué d'une tête polaire hydrophile orientée vers l'extérieur de la membrane et d'une queue hydrophobe orientée vers l'intérieur. Une membrane de cellule procaryote, de cellule eucaryote, animale ou végétale, ou d'un virus est décrite.

Une membrane vésiculaire, par exemple une membrane de VLP (Virus Like Particle), comme décrit par exemple dans les documents WO2007132790 **([29]),** WO1999036085 **([30]),** WO 2005058357 **([31]),** WO 2008092153 **([32]),** d'un lysosome, d'un exosome ou d'un proteoliposome, d'une membrane du réticulum endoplasmique, de l'appareil de Golgi, est décrite.

Une membrane d'une cellule eucaryote est décrite, avec une membrane vésiculaire, d'une membrane de réticulum endoplasmique rugueux, d'une membrane de l'appareil de Golgi, d'une membrane du réticulum endosplasmique lisse, ou d'une membrane plasmique, Une cellule hôte isolée transgénique issue d'une lignée cellulaire dans laquelle un ou plusieurs antigènes vaccinaux sont exprimés, par exemple par des techniques de génie génétique de l'ADN, ou de l'ARN recombinant, ou par infection d'une cellule par un vecteur viral exprimant un ou des antigènes vaccinaux d'intérêt est décrite**.** Toute technique de génie génétique connue de l'homme du métier permettant l'expression d'un transgène dans une cellule est décrite**.** Il peut s'agir d'une technique impliquant l'expression d'un ADN ou d'un ARN, par exemple un ARNm codant synthétique, introduit dans une cellule par transduction, par exemple par électroporation, micro-injection, ultra-sons, infection, transfection. L'expression peut être par exemple transitoire et/ou inductible et/ou constitutive d'au moins un antigène d'intérêt, comme par exemple décrit dans le document WO02090533 **([33])**. Toute cellule, notamment à l'exception des cellules souches embryonnaires humaines, par exemple une cellule humaine isolée - les cellules souches embryonnaires humaines étant par exemple exclues-, ou une cellule isolée animale - non-humaine- ou végétale. La cellule isolée peut être issue d'une lignée cellulaire choisie parmi Vero (ATCC No. CCL-81) comme Vero 76 (ATCC No. CRL-1587), CHO comme CHO-KI (CCL 61, ATCC), BHK comme BHK-21 [C-13] (ATCC® CCL-10™), HELA, perC6® (Crucell), HEK293 (ATCC® CRL-1573™), Sf9 (ATCC, CRL-1711), EBx® (Valneva / Vivalis, décrite dans le document WO2008129058 **([12])),** MDCK, par exemple MDCK (NBL-2) (ATCC® CCL-34™) **est** décrite**.** Dans le cas d'une cellule isolée d'une lignée cellulaire, on décrit les étapes suivantes :
- on inocule les cellules isolées avec un fluide contenant le virus,
- on réalise la propagation du virus dans les cellules,
- on collecte le virus ainsi propagé dans le surnageant de culture cellulaire,
- éventuellement on inactive le virus ainsi collecté,
- on réalise la fragmentation de la membrane des virus.

Dans le cas d'une cellule isolée d'une lignée cellulaire, le procédé suivant est décrit:
- on inocule les cellules isolées avec un fluide contenant le virus,
- on réalise la propagation du virus dans les cellules,
- on collecte les cellules exprimant le ou les antigènes à leur surface,
- on isole la membrane, par exemple la membrane plasmique, des cellules par une méthode de fractionnement membranaire, par exemple par cassage des cellules et centrifugations séquentielles,
- on réalise la fragmentation de la membrane.

Ou, encore alternativement :
- on exprime le ou les antigènes dans une cellule isolée par une méthode de génie génétique,
- on réalise l'amplification des cellules exprimant le ou les antigènes,
- on collecte les cellules exprimant le ou les antigènes à leur surface,
- on isole la membrane, par exemple la membrane plasmique, des cellules par une méthode de fractionnement membranaire, par exemple par cassage des cellules et centrifugations séquentielles,
- on réalise la fragmentation de la membrane.

Alternativement, une cellule issue d'un système allantoïque embryonné est décrite. A ce titre, ce système embryonné peut être de tout type d'espèce animale, par exemple un oeuf de poule, ou tout autre système allantoïque animal, notamment aviaire, permissif aux virus. Par exemple, pour l'obtention d'oeufs de poule embryonnés produisant une protéine membranaire telle que définie ci-dessus, les étapes peuvent être les suivantes :
- on inocule du virus, par exemple 200µL à une concentration de 10⁻³ pfu/mL, dans le liquide allantoïque d'oeufs embryonnés de 11 jours issus de poules élevées dans une enceinte stérile,
- ces oeufs sont ensuite incubés deux à trois jours à environ 33°C, au cours desquels le virus s'accumule dans le liquide allantoïque,
- on découpe les oeufs et on aspire le liquide allantoïque où la concentration en agents infectieux a été multipliée.

Dans le cas d'une membrane virale, il peut s'agir d'une enveloppe dérivée par bourgeonnement d'une membrane cellulaire de la cellule hôte infectée, cytoplasmique ou plasmique, selon les virus et/ou l'origine virale des antigènes vaccinaux exprimés. Dans ce cas, l'enveloppe est une membrane d'origine cytoplasmique ou plasmique de la cellule infectée, modifiée par l'expression de glycoprotéines virales associées à ces membranes lors du bourgeonnement viral, et les lipides de l'enveloppe sont, eux, d'origine cellulaire. Dans ce cas, il peut s'agir de la membrane du virus contre lequel une vaccination et/ou immunisation est souhaitée.

On entend par « calixarène», au sens de la présente invention, un surfactant supramoléculaire de la famille des calixarènes. A ce titre, les calixarènes utilisés dans l'invention peuvent être synthétisés par toute technique connue de l'homme du métier. Il peut s'agir par exemple d'un procédé tel que décrit dans le document WO2009/144419 **([28]).**

On entend par « alkyle », au sens de la présente invention, un radical carboné linéaire, ramifié ou cyclique, saturé ou insaturé, éventuellement substitué, comprenant 1 à 12 atomes de carbone. A titre indicatif, on peut citer les radicaux méthyle, éthyle, propyle, butyle, isobutyle, pentyle, hexyle, octyle, nonyle, décyle, undécyle, docécanyle et leurs isomères ramifiés. Le groupe alkyle peut éventuellement être substitué par un ou plusieurs hétéroatomes choisis dans le groupe O, S, N et P.

Le terme « aryle » désigne de manière générale un substituant aromatique cyclique comportant de 6 à 20 atomes de carbone. Dans le cadre de l'invention, le groupe aryle peut être mono- ou poly- cyclique éventuellement substitué. A ce titre, on peut citer le benzyle ou le phényle.

Dans le cadre de la présente invention, le terme « sels pharmaceutiquement acceptables » comprend les sels préparés avec des acides ou bases, non toxiques, en fonction des substituants présents sur les composés. Lorsque les composés de l'invention comportent des fonctions acides, les sels correspondants peuvent être obtenus par addition d'une base organique ou inorganique sur le composé sous forme neutralisée en présence éventuellement dans un solvant de préférence inerte. Des exemples de sel d'addition d'une base peuvent être les sels de sodium, potassium, calcium, ammonium, amino (organique), ou magnésium. Lorsque les composés de l'invention comportent des fonctions basiques, les sels correspondants peuvent être obtenus par addition d'un acide organique ou inorganique éventuellement dans un solvant de préférence inerte. Des exemples de sels d'additions d'acide inorganique peuvent être les sels hydrochlorique, hydrobromique, nitrique, carbonique, monohydrogéncarbonique, phosphorique, monohydrogénphosphorique, dihydrogénophosphorique, sulfurique, monohydrogénosulfurique hydroiodique. Des exemples de sels d'additions d'acide organique peuvent être les sels acétique, propionique, isobutyrique, maléique, malonique, benzoïque, succinique, subérique, fumarique, lactique, mandélique, phthalique, benzénesulfonique, p-tolylsulfonique, citrique, tartarique, méthanesulfonique.

**Une** étape de fragmentation réalisée avec au moins un calixarène de formule (I) est décrite dans laquelle :
- n est un nombre entier égal à 1 ;
- R¹, R², R³, R⁴ représentent, indépendamment l'un de l'autre, un atome d'hydrogène ; un groupe (C₁₋₁₈) alkyle linéaire ou ramifié ;
- X¹, X² et X³ représentent, indépendamment l'un de l'autre, un groupe -(CH₂)ₘ-COOR' ou -SO₃Y ou -(CH₂)ₘ-PO(OH)OY, ou-(CH₂)ₘ-NR'₃ dans lequel
   - m est un nombre entier allant de 0 à 20,
   - R' représente un atome d'hydrogène ;
ou l'un des sels pharmaceutiquement acceptables.

Un procédé de préparation d'un antigène vaccinal est décrit, comprenant une étape de fragmentation d'une membrane biologique associée audit antigène vaccinal par mise en contact de ladite membrane biologique avec au moins un calixarène de formule (III): 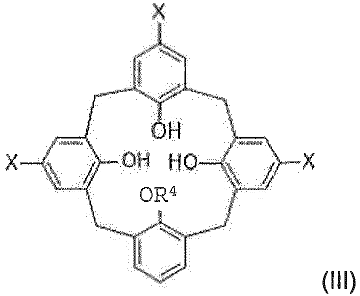 dans lequel :
- X est un groupement -(CH₂)ₒ(-CO₂Y,
- o est un nombre entier de 1 à 3,
- R⁴ est un groupe (C 1-4) alkyle linéaire ou ramidifé, et
- Y est un métal alcalin,
ou l'un de ses sels pharmaceutiquement acceptables,
dans lequel ledit antigène vaccinal obtenu comprend en outre un fragment de la membrane biologique associé à cet antigène.

Un procédé de préparation d'un antigène vaccinal est décrit**,** comprenant une étape de fragmentation d'une membrane biologique associée audit antigène vaccinal par mise en contact de ladite membrane biologique avec au moins un calixarène de formule (II) : 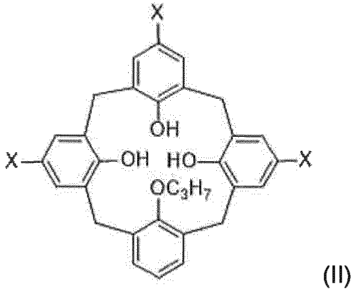 dans lequel :
- X est un groupement -(CH₂)ₒ-CO₂Y,
- o est un nombre entier de 1 à 3, et
- Y est un métal alcalin,
ou l'un de ses sels pharmaceutiquement acceptables,
dans lequel ledit antigène vaccinal obtenu comprend en outre un fragment de la membrane biologique associé à cet antigène sur laquelle l'étape de fragmentation est mise en oeuvre.

Selon un mode de réalisation particulier de l'invention, Il est décrit que o est égal à 1. Selon cet objet, le calixarène est appelé CALX133ACE.

Il est décrit que la mise en oeuvre, dans le procédé, des calixarènes, et notamment les calixarènes de formule (III) et notamment le CALX133ACE, permet une conservation optimale la conformation de l'antigène vaccinal, par exemple en conservant l'antigène vaccinal dans sa conformation au sein de la membrane virale qui est fragmentée. Avantageusement, le fait que l'antigène vaccinal obtenu par la mise en oeuvre du procédé de l'invention comprend un fragment de la membrane biologique associé à l'antigène vaccinal permet une conservation optimale de la conformation de l'antigène vaccinal. Avantageusement, la conservation de la conformation de l'antigène viral selon l'invention permet une meilleure efficacité, notamment vaccinale, de l'antigène viral fragmenté selon le procédé de l'invention, par rapport aux procédés de l'art antérieur fragmenté notamment au moyen de détergents.

On entend par « métal alcalin », au sens de la présente invention, tout métal alcalin, par exemple choisi parmi les sels de sodium, de potassium, d'ammonium, amino (organique) ou magnésium.

Dans un mode de réalisation particulier, o est égal à 1, et/ou Y est le sodium.

Avantageusement, les calixarènes utilisés dans la présente invention préservent la conformation des glycoprotéines membranaires telles que les antigènes vaccinaux de surface de manière améliorée par rapport aux autres surfactants, notamment le Triton X100 et le deoxycholate. Dans ce contexte, son utilisation permet de manière particulièrement avantageuse de générer des antigènes vaccinaux beaucoup plus efficaces d'un point de vue immunogénique et donc de réduire les quantités d'antigènes nécessaires dans les lots de vaccin pour une protection identique. De manière complémentaire, ces antigènes plus efficaces car mieux préservés, permettraient, dans un mode de réalisation particulier de l'invention, d'être une alternative, en tout ou partie, aux adjuvants.

On entend par « fragmentation d'une membrane biologique », au sens de la présente invention, toute décomplexification d'une membrane entière en fragments de membrane. Avantageusement, la fragmentation (également appelée « splitting ») d'une membrane biologique permet l'obtention de fragments de la membrane biologique comprenant les protéines membranaires. Avantageusement, le maintien de la protéine membranaire par le procédé de l'invention au sein d'un fragment de membrane biologique, ou en d'autres termes dans un contexte membranaire, permet une préservation de la conformation des protéines membranaires et une génération d'antigènes vaccinaux beaucoup plus efficaces d'un point de vue immunogénique que les méthodes de l'art antérieur.

L'étape de mise en contact peut être réalisée à un pH allant de 5,5 à 10, de préférence de 6 à 9, par exemple environ 7,95. L'étape de fragmentation peut être réalisée à une température allant de 0 à 100°C, de préférence de 4 à 25°C, notamment 4°C. L'étape de fragmentation peut mettre en oeuvre une concentration de calixarène, par exemple CALX133ACE allant de 10⁻⁶ à 10⁻² M.

L'étape de fragmentation peut être réalisée avec des calixarènes en solution ou des calixarènes en agrégats colloïdaux du fait de leur propriété tensioactive. Au sens de la présente invention, on entend par agrégat colloïdal des ensembles de quelques dizaines à quelques centaines de molécules de calixarène s'organisant en fonction des forces de répulsion vis-à-vis du solvant. Étant donné leur nature, les calixarènes, par exemple CALX133ACE, sont capables de former des agrégats dans un milieu approprié comme par exemple dans l'eau, dans une solution aqueuse, dans un milieu isotonique ou dans un milieu biologique.

L'agrégat peut être de préférence sous forme de micelles. Le terme micelle désigne est un agrégat sphéroïdal de molécules de calixarène de formule (I) qui s'organise en fonction du solvant utilisé. Par exemple dans l'eau ou un solvant aqueux, les extrémités lipophiles sont tournées vers l'intérieur de la micelle tandis que les extrémités hydrophiles forment l'interface de la micelle avec le solvant. Dans un solvant organique, par exemple de l'huile, l'arrangement est inversé. Le terme liposome désigne des petites vésicules fabriquées artificiellement constituées notamment de lamelles de phospholipides, séparées les unes des autres par des compartiments aqueux. Ils peuvent avoir une structure très proche de celle des membranes cellulaires. Dans le cadre de la présente invention, le terme nanoparticule signifie un assemblage de quelques centaines à quelques milliers de molécules de calixarène de formule (I), conduisant à un objet dont au moins l'une de ses dimensions est de taille nanométrique, par exemple entre 1 et 300 nm. Dans le procédé de l'invention, l'étape de mise en contact d'une suspension aqueuse comprenant la protéine membranaire à extraire avec le calixarène de formule (I) peut être éventuellement réalisée en présence d'au moins un co-soluté choisi dans le groupe comprenant :
i) les sels organiques et inorganiques choisi dans le groupe comprenant les sels pharmaceutiquement acceptables ;
ii) les petites molécules bioactives choisies dans le groupe des acides aminés, des vitamines, des lipides, des stéroïdes, des carbohydrates ou des métabolites ;
iii) les molécules bioactives oligomériques choisies dans le groupe des peptides, des oligonucléotides, des oligosaccharides ;
iv) les molécules biologiques polymériques choisies dans le groupe des protéines, polynucléotides et polysaccharides.

Selon un mode de réalisation de l'invention, l'étape de mise en contact peut être précédée par une étape dans laquelle :
- la protéine membranaire à extraire ou la fraction membranaire la contenant est solubilisée dans une solution tampon, et
- le calixarène de formule (I) est ajouté suivant les conditions de température, de pH et de concentration décrits précédemment.

Selon un mode de réalisation de l'invention, l'étape de mise en contact peut être suivie par une étape de centrifugation, permettant de séparer les protéines membranaires complexées avec les calixarènes de formule (I), des protéines membranaires non complexées.

Les protéines membranaires complexées avec les calixarènes de formule (I) et les protéines membranaires non complexées peuvent être séparées par centrifugation, par exemple pendant 1h, à 4° C et à une vitesse de 100 000g. Les conditions de centrifugation dépendront de la nature de la protéine. L'homme du métier saura choisir les conditions optimales de centrifugation, par exemple décrites dans Lenoir, G., Menguy, T., Corre, F., Montigny, C , Pedersen, P. A.,Thinès, D., le Maire, M., and Falson, P. (2002) Overproduction in yeast and rapid and efficient purification of the rabbit SERCAla Ca2+-ATPaSe, Biochim. Biophys. Acta 1560, 67-83 **([13]).**

Avantageusement, l'étape de fragmentation peut être précédée et/ou suivie d'une étape d'inactivation par toute méthode connue par l'homme du métier, par exemple au moyen de formaldéhyde, par la beta-propionolactone, par rayon ultra-violet, inactivation à la formaline, éventuellement suivie ou précédée par une étape de purification partielle, par exemple par ultracentrifugation, tel que décrit dans les documents US 2009/0060950 **([14])** et US6,048,537 **([15]).**

Le procédé de l'invention peut s'inscrire dans tout procédé classique de production industrielle d'antigène vaccinaux, par exemple les procédés décrits dans les documents WO 2011051235 **([16]),** US2011/0014230 **([17])**, WO 2002028426 **([18]),** US 2009/0060950 **([14]),** WO02/067983 **([9])**, US4,206,014 **([19]),** WO2009/115917 **([20]),** WO2005/113756 **([21]),** en remplaçant le composé utilisé pour la fragmentation (« splitting ») par les calixarènes de formule (I) ou un de ses sels.

Selon un mode de réalisation, une étape de diafiltration peut être réalisée. Avantageusement, elle permet d'éliminer le surfactant ou d'autres impuretés. Cette étape peut être réalisée par toute méthode connue de l'homme du métier. Il peut s'agir par exemple d'une technique choisie parmi une dialyse, une dilution, un échange de tampon sur colonne, une action enzymatique, une utilisation de polymères, par exemple Biobeads, notamment SM2 ou Dowex, une précipitation, ou une technique décrite par Ollivon et al. (Ollivon M1, Lesieur S, Grabielle-Madelmont C, Paternostre M. : « Vesicle reconstitution from lipid-detergent mixed micelles » Biochim Biophys Acta. 2000 Nov 23;1508(1-2):34-50 **([25]))**; cette liste n'étant pas exhaustive.

Avantageusement, une étape de filtration stérile peut être réalisée. Avantageusement, cette étape permet d'éliminer encore plus d'impuretés. Cette étape peut être réalisée par toute méthode connue de l'homme du métier. Il peut s'agir par exemple d'une filtration, par exemple à travers une membrane filtrante de porosité inférieure à 0,22 µm.

Dans un mode de réalisation, le procédé de l'invention peut comprendre en outre une étape de dialyse dudit antigène vaccinal. Avantageusement, cette étape permet d'éliminer encore plus d'impuretés. Cette étape peut être réalisée par toute méthode connue de l'homme du métier. Il peut s'agir par exemple d'une dialyse à travers une membrane de porosité inférieure à 14 000 Daltons.

Dans un mode de réalisation, le procédé peut comprendre en outre une étape de concentration de la protéine membranaire. Cette étape peut être réalisée par toute méthode connue de l'homme du métier. Il peut s'agir par exemple d'une précipitation, par exemple au PEG (polyéthylène glycol) ou sulfate d'ammonium, une évaporation, une utilisation de concentrateurs, par exemple de type Amicon®, par exemple Centricon® ou Centriprep® (Merck Millipore).

Le procédé de l'invention peut comprendre en outre toute étape connue de l'homme du métier dans la préparation de vaccins, par exemple décrite dans les procédés des documents WO 2011051235 **([16]),** US2011/0014230 **([17]),** WO 2002028426 **([18]),** US 2009/0060950 **([14]),** WO02/067983 **([9]),** US4,206,014 **([19]),** WO2009/115917 **([20]),** WO2005/113756 **([21]).**

Un antigène vaccinal susceptible d'être produit par la mise en oeuvre du procédé défini précédemment est également décrit.

Un vaccin comprenant un antigène vaccinal tel que décrit précédemment est également décrit. Le vaccin peut être administrable par toute voie connue et adaptée à la vaccination souhaitée, par exemple par voie injectable, notamment par voie sous-cutanée, intradermique, intra-musculaire, par voie orale, intranasale, transépithéliale, transdermique, patch, par comprimé muco-adhésif, voie rectale, par exemple par suppositoire.

Le vaccin peut être un vaccin avec adjuvant, du type Pandemrix® (GSK), Focetria® (Novartis) ou Humenza® (Sanofi Pasteur), ou sans adjuvant, du type Fluviral trivalent (GSK) ou Fluarix quadrivalent (GSK), de Celvapan® (Baxter) ou Vaxigrip® (Sanofi Pasteur). Dans ces exemples, le virus est remplacé par l'antigène vaccinal selon l'invention. Dans le cas d'un vaccin avec adjuvant, il peut s'agir de tout adjuvant connu de l'homme du métier permettant d'augmenter la réponse immunitaire, par exemple des sels d'aluminium, de la lysolécithine, des gels minéraux, de l'hydroxide d'aluminium, des microémulsions, des particules lipidiques, des oligonucléotides.

Le vaccin peut comprendre tout véhicule pharmaceutiquement acceptable, comme ceux contenus dans les vaccins mentionnés ci-dessus, ou encore des sels de stabilisation, des solubilisants, des osmo-régulateurs, des agents épaississants, des composés redox pour maintenir un potentiel redox physiologique.

Une dose de vaccin destiné à un être humain peut par exemple correspondre à un volume de 0,5 mL à injecter.

Le vaccin peut comprendre en outre un calixarène de formule (I) notamment sous format excipient. On entend par « excipient », au titre de la présente invention, une substance destinée à avoir un effet notoire. Il peut avantageusement conférer une conformation donnée, ou d'autres caractéristiques physiques particulières, au produit final, tout en évitant toute interaction non désirée, particulièrement chimique, avec le principe actif. A ce titre, la quantité de calixarène possiblement présente dans le vaccin peut être comprise de 0,1 à 1000µg, par exemple entre 0,5 et 700µg, ou entre 1 et 600µg ou encore entre 5 et 500 µg.

On décrit également l'utilisation d'un calixarène de formule (II) pour la préparation d'un vaccin ou d'un antigène vaccinal tel que décrit précédemment. L'utilisation d'un calixarène de formule (I) est également décrite.

Un autre objet de l'invention se rapporte à un antigène vaccinal ou un vaccin tel que défini précédemment, pour son utilisation comme médicament dans le traitement ou la prévention de la grippe Une maladie provoquée par un ou plusieurs des virus choisi parmi les suivants est décrite :
- la famille des virus orthomyxoviridae, incluant notamment les virus de la grippe, comme les les virus influenza de mamifère, et plus particulièrement les virus influenza humains, les virus influenza porcins, le virus influenza équin, le virus influenza félin, les virus influenza aviaires, le virus influenza du cygne ,
- la famille des virus paramyxoviridae, incluant notamment les respirovirus (sendai, virus parainfluenza bovin 3, parainfluenza 1 and 3 humain), les rubulaviruses (parainfluenza 2, 4, 4a, 4b humains, le virus humain des oreillons, parainfluenza de type 5), les avulavirus (virus de la maladie de Newcastle (NDV)), les pneumoviruses (virus respiratoires syncytiaux humain et bovin), les métapneumovirus (métapneumovirus animal et humain), les morbilivirus (virus de la rougeole, de la maladie de carré et de la peste bovine) et les henipavirus (virus hendra, nipah...etc),
- la famille des virus coronaviridae incluant noatmment les coronavirus humains (notamment NL63, SARS-CoV, MERS-CoV) et animaux (coronavirus canin, porcin, bovin et de la bronchite infectieuse aviaire) ;
- la famille des virus flaviviridae incluant notamment les arbovirus (virus de l'encéphalite à tiques), les flavivirus (virus de la dengue, de la fièvre jaune, de l'encéphalite de Saint Louis, de l'encéphalite japonaise, du Nil occidental incluant le sous type Kunjin, de la Muray vallée, virus Rocio, virus Ilheus, virus de la méningo-encéphalite à tique), les hepacivirus (virus de l'hépatite C, le virus de l'hépatite A, le virus de l'hépatite B) et les pestivirus (virus de la maladie des frontières, de la diarrhée bovine, de la fièvre du cygne),
- la famille des virus Rhabdoviridae incluant notamment les vésiculovirus (virus de la stomatite vésiculaire), les lyssavirus (virus de la rage, des chauve-souris de Lagos, australienne, européenne), les ephemerovirus (virus de la fièvre éphémère bovine), les novirhabdovirus (virus de la tête de serpent, de la septicémie hémorragique et de la nécrose hématopoïétique),
- la famille des virus Togaviridae incluant notamment les rubivirus (virus de la rubéole), les alphavirus (notamment le virus Sinbis, virus Semliki forest, virus O'nyong'nyong, virus Chikungunya, virus Mayaro, virus de Ross river, virus de l'encéphalite équine de l'Est, virus de l'encéphalite équine de l'Ouest, virus de l'encéphalite équine du Venezuela),
- la famille des virus herpesviridae incluant notamment les herpesvirus humain (HSV-1, HSV-2, le vius de la varicelle, le virus Epstein-Barr, cytomégalovirus, roséolovirus, HHV-7 et le KSHV),
- la famille des virus poxviridae incluant notamment les orthopoxvirus (comme notamment camoepox, cowpox, smallpox, vaccinia), les carpipoxvirus (incluant notamment sheepox), les avipoxvirus (incluant notamment le fowlpox), les parapoxvirus (incluant notamment le virus de la stomatite papuleuse bovine) et les leporipoxvirus (incluant notamment le virus de la myxomatose),
- la famille des virus rétroviridae incluant notamment les lentivirus (incluant notamment les virus de l'immunodéficience humaine 1 et 2, féline, simienne, le virus de l'arthrite encéphalite caprine ou de la maladie de Maedi-Visna) et les rétrovirus (incluant notamment les virus du sarcome de Roux, lymphotrophique humains 1, 2 et 3).

Par exemple, dans le cas du virus de la grippe, le vaccin peut comprendre un ou plusieurs sous-types de virus influenza A choisis parmi les sous-types humains de H1N1, H2N2, H3N2, H5N1, H7N7, H7N9, H1N2, H9N2, H7N2, H7N3, H10N7, les sous-types de la grippe porcine de H1N1, H1N2, H3N1 et H3N2, les sous-type de la grippe canine et de la grippe équine H7N7, H3N8 ou les sous-types de la grippe aviaire H5N1, H7N2, H1N7, H7N3, H13N6, H5N9, H11N6, H3N8, H9N2, H5N2, H4N8, H10N7, H2N2, H8N4, H14N5, H6N5, H12N5, ou tout autre virus réassortant humain et/ou animal. Alternativement, dans le cas du virus de la grippe, le vaccin peut comprendre un ou plusieurs sous-types de virus influenza B humains choisis, par exemple un lignage choisi parmi le lignage Victoria et le lignage Yamagata. Dans le cas du virus influenza B, il peut s'agir notamment de la souche vaccinale B/ Massachussetts/2/2012 ou B/Brisbane/33/2008.

Un autre objet décrit se rapporte à l'utilisation d'un calixarène tel que défini ci-avant, comme adjuvant dans un vaccin.

Les calixarènes définis ci-avant peuvent être utilisés pour leur activité immunologique, et plus particulièrement immunomodulatrice. Ils peuvent, selon le sujet, les doses, l'antigène et le moment où il est administré, provoquer une immunostimulation.

On entend par « adjuvant », au sens de la présente invention, une substance qui, quand elle est administrée (avalée, inhalée, injectée, etc.) conjointement avec un antigène, stimule, active, prolonge, renforce ou module le système immunitaire, bien que cette substance n'ait pas elle-même de vertu antigénique.

A ce titre, la quantité de calixarène possiblement présente dans le vaccin peut être comprise de 0,1 à 1000µg, par exemple entre 0,5 et 700µg, ou entre 1 et 600µg ou encore entre 5 et 500 µg dans le au poids total du vaccin.

Dans un mode de réalisation il est décrit l'adjuvant peut être ajouté à tout vaccin, par exemple à un vaccin inactivé ou atténué, ou un vaccin fragmenté, quel que soit le procédé de production utilisé.

D'autres avantages pourront encore apparaître à l'homme du métier à la lecture des exemples ci-dessous, illustrés par les figures annexées, donnés à titre illustratif.

### Brève description des figures

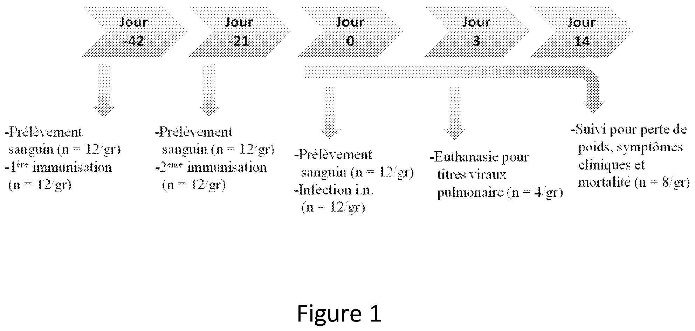

- La figure 1 représente le protocole d'immunisation CALX133ACE avec chalenge chez la souris, à J-42 (42 jours avant l'infection), J-21 (21 jour avant l'infection), J0 (jour de l'infection et du prélèvement sanguin), J4 (4 jours après l'infection) et J14 (14 jours après l'infection).

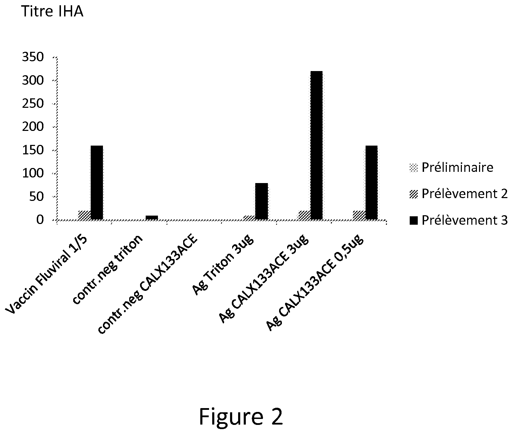

- La figure 2 représente la mesure du titre IHA (inhibition d'hémagglutination) à J-42 (« Préliminaire »), J-21 (« Prélèvement 2 ») et J0 (« Prélèvement 3), de souris infectées par H1N1 pdm (10^{e}3) et éventuellement immunisées par (de gauche à droite) : vaccin Fluviral® 1/5, contrôle négatif Triton X100, contrôle négatif CALX133ACE, antigène 3µg Triton X100, antigène 3µg - 1 dose Triton X100, antigène 3µg CALX133ACE et antigène 0,5µg CALX133ACE.

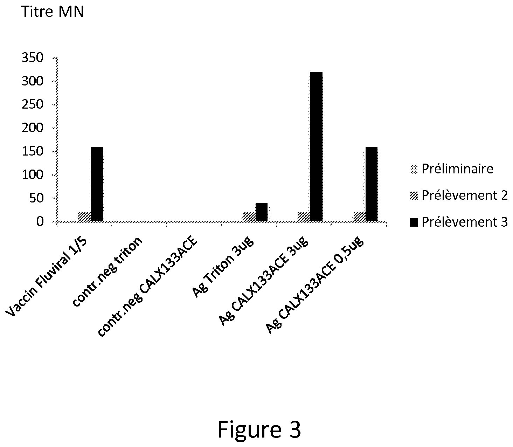

- La figure 3 représente la mesure du titre MN (microneutralisation) à J-42 (« Préliminaire »), J-21 (« Prélèvement 2 ») et J0 (« Prélèvement 3), de souris infectées par H1N1 pdm (10^{e}3) et éventuellement immunisées par (de gauche à droite) : vaccin Fluviral® 1/5, contrôle négatif Triton X100, contrôle négatif CALX133ACE, antigène 3µg Triton X100, antigène 3µg - 1 dose Triton X100, antigène CALX133ACE 3µg et antigène CALX133ACE 0,5µg.

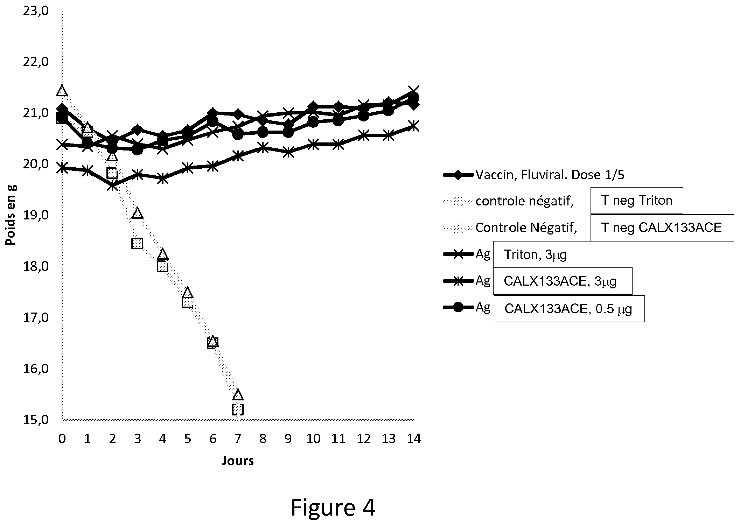

- La figure 4 représente le suivi de poids (en g) des souris immunisées et infectées avec H1N1 pdm (10^{e}3 IU), de J0 à J14, pour les souris auxquelles ont été administrées : le vaccin Fluviral®, dose 1/5 (losanges), le contrôle négatif T. Neg. Triton X100 (carrés), le contrôle négatif T Neg CALX133ACE (triangles), l'antigène 3µg extrait au Triton X100 (croix), l'antigène 3µg extrait au CALX133ACE (étoile), et l'antigène 0,5µg extrait au CALX133ACE (rond).

### EXEMPLES

### Exemple 1 : Protocole d'extraction des antigènes vaccinaux influenza utilisant les molécules CALX133ACE et CALX1103ACE

On évalue les molécules CALX133ACE et CALX1103ACE (CALX1103ACE étant un calixarène dans lequel R⁴ est un groupe alkyle en C10) pour leur capacité à extraire les antigènes vaccinaux influenza (produite à partir d'une production de la semence vaccinale X-179a, H1N1 pandémique 2009) et on les compare avec un détergent utilisé par les producteurs de vaccins (Triton X100) et deux détergents commerciaux (FC12 et C12E8). Plusieurs conditions de concentration, de pH et de température d'extraction ont été évaluées.

L'évaluation quantitative et qualitative de l'extraction des antigènes vaccinaux a été réalisée par les tests de référence industrielle de mesure d'activité hémagglutinante (test d'hémagglutination : Hirst, G.K. 1942. The quantitative détermination of influenza virus and antibodies by means of red cell agglutination. J. Exp. Med. 75:47-64 **([22])** et Salk, J.E. 1944. Simplified procédure for titrating hemagglutinating capacity of influenza virus and the corresponding antibody. J. Immunol. 49:87-98 **([23]))** et de quantification (SRID ; A quantitative, single-radial-diffusion test for immunological studies with influenza virus. Schild GC, Henry-Aymard M, Pereira HG. J Gen Virol. 1972 Aug;16(2):231-6 **([24]))** des antigènes extraits.

Les conditions testées ont été celles mentionnées dans le Tableau I:

**Tableau I:**

| N° condition | Molécule testée | Concentration | Température | pH tampon PO₄ | Temps de contact |
|---|---|---|---|---|---|
| 1 | Triton X100 | 4% | ambiante | 7,95 | 1h |
| 2 | Triton X100 | 4% | 37°C | 7,95 | 1h |
| 3 | CALX133ACE | 1% | ambiante | 7,95 | 1h |
| 4 | CALX133ACE | 1% | 37°C | 7,95 | 1h |
| 5 | CALX1103ACE | 1% | ambiante | 7,95 | 1h |
| 6 | CALX1103ACE | 1% | 37°C | 7,95 | 1h |
| 7 | CALX133ACE | 1% | 4°C | 7,95 | 2h |
| 8 | CALX1103ACE | 1% | 4°C | 7,95 | 2h |
| 9 | Triton X100 | 4% | 37°C | 7,95 | 1h |
| 10 | Triton X100 | 4% | 37°C | 6,74 | 1h |

Après extraction, une dialyse des antigènes fragmentés (surnageant SN et culot d'extraction) contre du PBS 1 X a été menée dans le procédé classique de production industrielle (par exemple [9], [18]) afin de diminuer la concentration en sels et détergents des produits d'extraction. Chaque lot a donc été dialysé. Il a été déterminé que le Triton X100 n'était pas complètement éliminé lors de la dialyse. Cependant, il est apparu que la molécule CALX133ACE pouvait être dialysée, suggérant qu'elle pourrait n'être présente qu'à l'état de trace dans un produit vaccin éventuel commercialisé.

Après dialyse, les produits d'extraction ont été aliquotés et titrés pour leur activité hémagglutinante (HA). Les données sont présentées dans le Tableau II suivant :

**Tableau II**

| N° condition | Molécule testée | Concentration | Température | pH tampon PO₄ | Temps de contact | Titre en UHA/50µL | |
|---|---|---|---|---|---|---|---|
| | | | | | | surnageant | culot |
| 10 | Triton X100 | 4% | 37°C | 6,74 | 1h | 128 | 16 |
| 1 | Triton X100 | 4% | ambiante | 7,95 | 1h | 128 | 64 |
| 2 | Triton X100 | 4% | 37°C | 7,95 | 1h | 128 | 16 |
| B | CALX133ACE | 0,1% | 4°C | 7,95 | 2h | 128 | 4096 |
| 7 | CALX133ACE | 1% | 4°C | 7,95 | 2h | 512 | 2 |
| 3 | CALX133ACE | 1% | Ambiante | 7,95 | 1h | 512 | 4 |
| 4 | CALX133ACE | 1% | 37°C | 7,95 | 1h | 128 | 0 |
| D | CALX1103ACE | 0,1% | 4°C | 7,95 | 2h | 64 | 128 |
| 8 | CALX1103ACE | 1% | 4°C | 7,95 | 2h | 1024 | 32 |
| 5 | CALX1103ACE | 1% | Ambiante | 7,95 | 1h | 512 | 32 |
| 6 | CALX1103ACE | 1% | 37°C | 7,95 | 1h | 512 | 16 |

Les résultats indiquent des titres hémagglutinants (HA) significatifs (512 et 1024 respectivement) pour les antigènes fragmentés (SN) obtenus après extraction avec les molécules CALX133ACE et CALX1103ACE à la concentration de 1% dans des conditions à 4°C et à pH=7,95. Ces titres sont significativement supérieurs à ceux obtenus pour les antigènes extraits (SN) suivant le protocole classique utilisant du Triton X100.

Ces résultats sont d'autant plus significatifs que (i) le titre hémagglutinant obtenu est dépendant de la concentration utilisée des molécules CALX133ACE (0,1% vs 1%) et (ii) que le titre HA des reliquats d'antigènes non extraits (culot) est inversement proportionnel et inférieur à celui obtenu avec le Triton X100. La température d'extraction apparaît être un paramètre important ; la température de 4 °C semble être la température optimale d'extraction des antigènes vaccinaux par les molécules CALX133ACE et CALX1103ACE.

Ces premiers résultats ont indiqué clairement que les molécules CALX133ACE étaient adaptées à l'extraction des antigènes vaccinaux influenza à partir de lots de virus produits dans le système allantoïque de production utilisé par les industriels du vaccin. Ces molécules CALX133ACE et CALX1103ACE permettent une extraction qui est plus efficace que celle classique utilisant le Triton X100, d'un point de vue qualitatif (plus d'antigènes extraits, activité hémagglutinante supérieure, profil natif d'oligomérisation des antigènes).

### Exemple 2: Optimisation de la fragmentation des antigènes vaccinaux et leur quantification pour la réalisation d'un test d'efficacité en modèle murin:

La production virale a été réalisée par Inoculation d'oeufs de poule embryonnés de 11 jours avec des inoculum viraux (semence vaccinale) puis concentrée et purifiée. La fragmentation a été réalisée suivant la méthode classique industrielle utilisant le Triton X100 (4%), en comparaison de l'utilisation de molécules CALX133ACE.

L'évaluation qualitative de l'extraction des antigènes vaccinaux est réalisée par le test de référence industrielle qui est la mesure de leur activité hémagglutinante (activité de l'antigène principal, l'hémagglutinine en titre UHA/50 ml). L'évaluation quantitative est réalisée par la technique de référence industrielle, également utilisée par les autorités du médicament pour la validation et l'autorisation de mise sur le marché des lots de vaccins: Single Radial ImmunoDiffusion (SRID, quantité de HA en mg/ml). Enfin, la quantification de HA par spectrométrie de masse iTRAQ permet d'obtenir une quantification relative entre les différents échantillons à comparer en test animal (quantité de HA en mg/ml).

Les molécules et conditions qui ont été choisies pour la fragmentation ont été:
- Le Triton X100 à 4% pendant 1h à 37°C
- La molécule CALX133ACE à 1% pendant 2h à 4°C

Les molécules CALX133ACE n'entrainent pas d'hémolyse des globules rouges en absence d'antigènes grippaux lors du test d'hémagglutination. De plus, ces molécules sont dialysables, ce qui implique que le CALX133ACE pourra être limité à l'état de trace dans l'échantillon.

### Exemple 3 : Expérimentation animale

Dans une première expérience, il a été réalisé plusieurs lots d'antigènes dans les conditions optimales de production décrites précédemment, utilisant le Triton X100 ou la molécule CALX133ACE comme agent de splitting. Le Tableau III ci-dessous est un bilan des matériels fragmentés utilisés pour le test animal.

**Tableau III:**

| Condition expérimentale | % détergent résiduel | Volume approximatif d'échantillon (µL) | Titre en UHA/50µL | Concentration HA (µg/mL) par quantif SRID | Rapport ITRAQ | [HA] corrigée par ITRAQ (µg/mL) | Volume à injecter par souris (µL) | |
|---|---|---|---|---|---|---|---|---|
| | | | | | | | Pour 0,5µg d'Ag HA | Pour 2,5 µg d'Ag HA |
| Split Triton X100 4% | 0,36 | 900 | 1024 | 144,83 | Split Triton X100/split CALX133A CE C=1,43 | 144,83 | 3,45 | 17,26 |
| Split CALX133ACE 1% | 0,03 | 800 | 4096 | 54,08 | | 101,28 | 4,94 | 24,68 |
| Surnageant Triton X100 4% | 0,19 | 1000 | 1024 | 119,21 | Surnageant Triton X100/S CALX133A CE C=0,93 | 119,21 | | 20,97 |
| Surnageant CALX133ACE 1% | 0,04 | 850 | 1024 | 48,41 | | 128,64 | | 19,43 |

Dans cette première expérience, il a été prévu de tester deux quantités d'antigènes HA pour les splits (0,5 et 2,5µg) et une seule pour les surnageants obtenus par ultracentrifugation des splits. Pour chaque condition, 12 souris Balb.c ont été immunisées deux fois (à J0 puis à J21) par voie intramusculaire (100µL total). Plusieurs prélèvements sanguins des animaux ont été effectués à J0, J21, J42 et J63 (Ponctions cardiaques et euthanasie). Après chaque série de prélèvement sanguin, les sérums d'un même groupe ont été récoltés et mélangés pour avoir suffisamment de matériel permettant de réaliser un test d'inhibition d'hémagglutination (IHA) en duplicata et un test de microneutralisation en duplicata également.

Les résultats obtenus sont présentés dans le Tableau IV suivant :

**Tableau IV:**

| | Préliminaire | | Prélèvement 2 | | Prélèvement 3 | | Ponction cardiaque | |
|---|---|---|---|---|---|---|---|---|
| | IHA | Microneutralisation (MN) | IHA | Microneutralisation (MN) | IHA | Microneutralisation (MN) | IHA (moy) | Microneutralisation (MN) |
| Groupe 1 split Triton X100 0,5 µg d'Ag | <10 | <10 | <10 | <10 | <10 | 20 | <10 | <10 |
| Groupe 2 split Triton X100 2,5 µg d'Ag | <10 | <10 | <10 | <10 | 80 | 80 | 29,17 | 40g |
| Groupe 3 split CALX133ACE 0,5 µg d'Ag | <10 | <10 | <10 | <10 | 40 | 80 | 25,83 | 40 |
| Groupe 4 split CALX133ACE 2,5 µg d'Ag | <10 | <10 | <10 | <10 | 80 | 160 | 36,67 | 80 |
| Groupe 5 surnageant Triton X100 2,5 µg | <10 | <10 | <10 | <10 | 40 | 40 | 19,17 | 40 |
| Groupe 6 surnageant CALX133ACE 2,5 µg | <10 | <10 | <10 | <10 | <10 | 20 | 0,83 | 20 |

L'expérimentation avec des antigènes générés par fragmentation CALX133ACE a été validée (bonne tolérance des souris, qualité et quantité des sérums prélevés, robustesse des résultats d'IHA avec contrôles (+) et (-) validés).

Les résultats IHA et de microneutralisation sont cohérents et indiquent que le split d'antigènes généré par fragmentation CALX133ACE induisent une réponse anticorps significative (prélèvement 3 et ponction cardiaque), à la différence des antigènes purifiés par ultracentrifugation des splits (noté « surnageant » dans les tableaux III et IV), car ces antigènes purifiés ne sont plus dans un contexte membranaire, ce qui valide le procédé de production et de fragmentation des antigènes vaccinaux par la molécule CALX133ACE en termes qualitatif et quantitatif.

De plus, il apparaît une différence significative en faveur du procédé CALX133ACE vis à vis du procédé Triton X100 en ce qui concerne la dose injectée à 0,5 µg (prélèvement 3 et ponction cardiaque). La même tendance est retrouvée avec la dose de 2,5 µg, mais la différence d'IHA n'est pas significative (prélèvement 3 et ponction cardiaque).

De plus, il apparaît que la réponse anticorps induite par une dose de 0,5 µg d'antigènes générés par le procédé CALX133ACE est similaire à celle induite avec 2,5 µg d'antigènes générés par le procédé Triton X100 (prélèvement 3 et ponction cardiaque). Ce dernier résultat est important pour l'invention car il souligne la possibilité d'obtenir une réponse anticorps avec 5 fois moins d'antigènes lorsqu'ils sont générés avec la molécule CALX133ACE.

### Exemple 4: Immunisation de souris avec le CALX133ACE : évaluation chez la souris du pouvoir protecteur des antigènes vaccinaux générés

Dans une seconde expérience, il a été réalisé plusieurs lots d'antigènes dans les conditions optimales de production décrites précédemment, utilisant le Triton X100 ou la molécule CALX133ACE comme agent de splitting. Le Tableau V ci-dessous est un bilan des matériels fragmentés utilisés pour cette seconde expérience animale. Il a été prévu d'immuniser 2 fois des souris Balb/c seront avec les préparations antigéniques CALX133ACE ou Triton X100 et ensuite de les infecter avec la souche A/H1N1 California/7/2009 (H1N1 pdm09) afin de vérifier la protection chez l'animal.
**Groupe 1** : 12 souris (contrôle positif) immunisées i.m. (intra-musculaire) avec 1/5 d'une dose de Fluviral® (correspondant à 3 ug de HA par antigène).
**Groupe 2:** 12 souris contrôle négatif (« Ctrl ») ) immunisées i.m. avec la procédure de Triton X100 sans antigène (6 souris) ou de CALX133ACE sans antigène (6 souris).
**Groupe 3**: 12 souris immunisées i.m. avec 3 ug de la préparation HA avec Triton X100.
**Groupe 4:** 12 souris immunisées i.m. avec 3 ug de la préparation HA avec CALX133ACE.
**Groupe 5:** 12 souris immunisées i.m. avec 0,5 ug de la préparation HA avec CALX133ACE.

Le protocole d'immunisation est représenté sur la Figure 1.

Le Tableau V ci-dessous est un bilan des matériels fragmentés utilisés pour ce test.

**Tableau V :**

| Condition expérimentale | % de détergent dans l'échantillon brut | % de détergent après ajustement | % de détergent injecté à la souris | Volume approximatif d'échantillon (µL) | Titre HA (UHA/50µ L) | [HA] par SRID (µg/mL) | Rapport ITRAQ | [HA] corrigée par ITRAQ (µg/mL) | Volume à injecter par souris (µL) | |
|---|---|---|---|---|---|---|---|---|---|---|
| | | | | | | | | | Pour 0,5 µg d'Ag | Pour 3 µg d'Ag |
| Split Triton X100 4% | 0,15 | 0,22 | 0,120 | 1650 | 4096 | 108,88 | Split CALX13 3ACE /Split Triton X100 T=0,61 | 108,88 | | 27,55 |
| Split CALX133ACE 1% | 0,03 | 0,03 | 0,004 ou 0,022 | 3500 | 16384 | 68,37 | | 82,30 | 6,08 | 36,45 |
| Ctrl -Triton X100 4% | 2 | 0,22 | 0,120 | 850 | - | - | - | - | | 27,55 |
| Ctrl - CALX133ACE 1% | 0,02 | 0,03 | 0,022 | 670 | - | - | - | - | | 36,45 |

Les souris ont été sacrifiées à J+4 (pic de réplication du virus), avec mesure de leur titre viral pulmonaire.

Le titre viral pulmonaire des souris immunisées et infectées à J+4 avec H1N1 pdm09 (10^{e}3 IU), a été mesuré après euthanasie et indiqué dans le Tableau VI ci-dessous.

**Tableau VI :**

| | | | Titre pulmonaire | | |
|---|---|---|---|---|---|
| | Souris | | log/ml | pfu/ml | pfu/g |
| Vaccin Fluviral® 1/5 | 1-9 | | <10e2 | | |
| | 1-10 | | <10e2 | | |
| | 1-11 | | <10e2 | | |
| | 1-12 | | <10e2 | | |
| Ctrl Triton X100 | 2-4 | | 9 | 9,3x10e8 | 4,88x10e9 |
| | 2-6 | | 8,5 | 2,9x10e8 | 1,71x10e9 |
| Ctrl CALX133ACE | 2-7 | | 7,7 | 5,4x10e7 | 3,7x10e8 |
| | 2-11 | | 8,6 | 4,1x10e8 | 2,09x10e9 |
| Split Triton X100 3µg d'Ag | 3-1 | | <10e2 | | |
| | 3-11* | | 4,4 | 2,6x10e4 | 1,71x10e5 |
| | 3-12 | | <10e2 | | |
| Split CALX133ACE 3µg d'Ag | 4-7 | | <10e2 | | |
| | 4-8 | | <10e2 | | |
| | 4-9 | | <10e2 | | |
| | 4-10 | | <10e2 | | |
| Split CALX133ACE 0,5µg d'Ag | 5-6 | | <10e2 | | |
| | 5-7 | | <10e2 | | |
| | 5-8 | | <10e2 | | |
| | 5-9 | | <10e2 | | |

| | | | | | |
|---|---|---|---|---|---|
| * 1 seule immunisation | | | | | |

Un suivi du poids des souris Balbc a été mené après infection intranasale par 10^{e}3 IU/souris de virus influenza A H1N1 pdm09 (pandémique) et est décrit dans la Figure 4.

### Mesure de la protection par IHA

Les groupes suivants de souris sont testés :
- souris immunisées avec le vaccin Fluviral® 1/5
- souris immunisées avec le Triton X100 uniquement
- souris immunisées avec du CALX133ACE
- souris immunisées avec l'antigène viral préparé avec du Triton X100 3µg
- souris immunisées avec l'antigène vaccinal extrait par CALX133ACE 3 µg
- souris immunisées avec l'antigène vaccinal extrait par CALX133ACE 0,5 µg

Ces souris sont infectées avec H1N1 pdm (10^{e}3).

Les résultats du test IHA sur les sérums des souris immunisés et infectées avec H1N1 pdm (10^{e}3) sont donnés dans le Tableau VI.

**Tableau VII :**

| | | | A/Californ ia/7/2009 | | |
|---|---|---|---|---|---|
| | | Nombre de souris | Préliminaire | Prélèvement 2 | Prélèvement 3 |
| Groupe 1 | Vaccin Fluviral® 1/5 | 12 | <10 | 20 | 160 |
| Groupe 2-1 | Ctrl Triton X100 | 6 | <10 | <10 | 10 |
| Groupe 2-2 | Ctrl CALX133ACE | 6 | <10 | <10 | <10 |
| Groupe 3-1 | Split Triton X100 3µg d'Ag | 10 | <10 | 10 | 80 |
| Groupe 4 | Split CALX133ACE 3µg d'Ag | 12 | <10 | 20 | 320 |
| Groupe 5 | Split CALX133ACE 0,5µg d'Ag | 12 | <10 | 20 | 160 |
| Contrôle + | (A/California) | | 1280 | | |
| Contrôle - | (A/Perth) | | <10 | | |
| | | | J-42 | J-21 | J0 |

La mesure de la protection par IHA est montrée sur la Figure 2.

### Mesure de la protection par MN (microneutralisation)

Les groupes suivants de souris sont testés :
- souris immunisées avec le vaccin Fluviral® 1/5
- souris immunisées avec le Triton X100 uniquement
- souris immunisées avec du CALX133ACE
- souris immunisées avec l'antigène viral préparé avec du Triton X100 3µg
- souris immunisées avec l'antigène vaccinal extrait par CALX133ACE 3 µg
- souris immunisées avec l'antigène vaccinal extrait par CALX133ACE 0,5 µg

Ces souris sont infectées avec H1N1 pdm (10³).

Les tests MN sur les sérums des souris imumnisées et infectées avec H1N1 pdm (10³) sont résumés dans le Tableau VIII ci-dessous.

**Tableau VIII:**

| | | | 17/06/2014 | | |
|---|---|---|---|---|---|
| | | | A/Californ ia/7/2009 | | |
| | | Nombre de souris | Préliminaire | Prélèvement 2 | Prélèvement 3 |
| Groupe 1 | Vaccin Fluviral 1/5 | 12 | <10 | 20 | 160 |
| Groupe 2-1 | Ctrl Triton X100 | 6 | <10 | <10 | <10 |
| Groupe 2-2 | Ctrl CALX133ACE | 6 | <10 | <10 | <10 |
| Groupe 3-1 | Split Triton X100 3µg d'Ag | 10 | <10 | 20 | 40 |
| Groupe 4 | Split CALX133ACE 3µg d'Ag | 12 | <10 | 20 | 320 |
| Groupe 5 | Split CALX133ACE 0,5µg d'Ag | 12 | <10 | 20 | 160 |
| | | | J-42 | J-21 | J0 |

La mesure de la protection par MN est montrée sur la Figure 3.

### Protection des souris

La figure 5 représente le suivi de poids des souris immunisées et infectées avec H1N1 pdm (10³ IU).

Dans tous les groupes immunisés (Fluviral, Triton X100 et CALX133ACE): aucune perte de poids ni de mortalité.

Seules les souris dans les 2 groupes contrôles négatifs sont mortes.

### Conclusion

### Tests IHA et MN:

L'immunisation est aussi efficace avec 0,5 µg de split d'antigène/CALX133ACE qu'avec 3 µg de Fluviral®.

L'immunisation est plus efficace avec 3 µg de split d'antigène CALX133ACE qu'avec 3 µg de split d'antigène/Triton X100.

### Protection:

La protection est aussi efficace avec 0,5 µg de split d'antigène CALX133ACE qu'avec 3 µg Fluviral ou 3 µg de split d'antigène/Triton X100.

### Listes des références

1. Barrett PN, Portsmouth D, Ehrlich HJ. Developing cell culture-derived pandemic vaccines. Curr Opin Mol Ther. 2010 Feb;12(1):21-30.
2. Le Ru A, Jacob D, Transfiguracion J, Ansorge S, Henry O, Kamen AA. Scalable production of influenza virus in HEK-293 cells for efficient vaccine manufacturing. Vaccine. 2010 May 7;28(21):3661-71.
3. FR 10/55716.
4. WO 2012007380.
5. FR 10/59132.
6. WO 2012059696.
7. Ellebedy AH, Webby RJ. Influenza vaccines. Vaccine. 2009 Nov 5;27 Suppl 4:D65-8.
8. WO02/28422.
9. WO02/067983.
10. M. Rosen, « Surfactants and Interfacial Phenomena », 3rd Ed., Hoboken: John Wiley & Sons, Inc., 2004.
11. A.M. Schwartz, J.W. Perry, J. Berch, « Surface Active Agents and Detergents », vol II, R. Krieger Pub. Co., New York, 197.
12. WO2008129058.
13. Lenoir, G., Menguy, T., Corre, F., Montigny, C , Pedersen, P. A.,Thinès, D., le Maire, M., and Falson, P. (2002) Overproduction in yeast and rapid and efficient purification of the rabbit SERCAla Ca2+-ATPaSe, Biochim. Biophys. Acta 1560, 67-83.
14. US 2009/0060950.
15. US6,048,537.
16. WO 2011051235.
17. US2011/0014230.
18. WO 2002028426.
19. US4,206,014.
20. WO2009/115917.
21. WO2005/113756.
22. Hirst, G.K. : "The quantitative determination of influenza virus and antibodies by means of red cell agglutination". J. Exp. Med. 75:47-64. 1942.
23. Salk, J.E.: "Simplified procédure for titrating hemagglutinating capacity of influenza virus and the corresponding antibody. J. Immunol. 49:87-98. 1944".
24. Schild GC, Henry-Aymard M, Pereira HG. J : "SRID ; A quantitative, single-radial-diffusion test for immunological studies with influenza virus". Gen Virol. 1972 Aug;16(2):231-6.
25. Ollivon M1, Lesieur S, Grabielle-Madelmont C, Paternostre M. : « Vesicle reconstitution from lipid-detergent mixed micelles » Biochim Biophys Acta. 2000 Nov 23;1508(1-2):34-50.
26. WO 2010/058100.
27. WO2010/058099.
28. WO2009/144419.
29. WO2007132790.
30. WO1999036085.
31. WO 2005058357.
32. WO 2008092153.
33. WO02090533.

## Revendications

1. Procédé de préparation d'un antigène vaccinal fragmenté du virus de la grippe, comprenant une étape de fragmentation d'une membrane biologique associée audit antigène vaccinal par mise en contact de ladite membrane biologique avec au moins un calixarène de formule (II) : 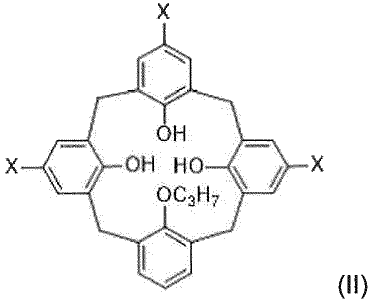 dans lequel :
- X est un groupement -(CH₂)-CO₂Y, et
- Y est un métal alcalin choisi parmi les sels de sodium, de potassium, d'ammonium, amino (organique) ou magnésium,
ou l'un de ses sels pharmaceutiquement acceptables,
dans lequel ladite étape de fragmentation est réalisée à une température allant de 4 à 25°C, et à une concentration dudit calixarène de 1%.

2. Procédé selon la revendication 1, dans lequel ledit virus de la grippe est choisi parmi les virus influenza A et les virus influenza B.

3. Procédé selon l'une quelconque des revendications précédentes, comprenant en outre une étape de dialyse dudit antigène vaccinal.

4. Vaccin contre le virus de la grippe susceptible d'être produit par la mise en oeuvre du procédé défini dans l'une quelconque des revendications 1 à 3, comprenant un calixarène de formule (II) sous format excipient en une quantité comprise de 0,1 à 1000µg dans le poids total du vaccin.

5. Vaccin selon la revendication 4, dans lequel ledit antigène vaccinal comprend une protéine membranaire d'origine virale.

6. Utilisation d'un calixarène de formule (II): 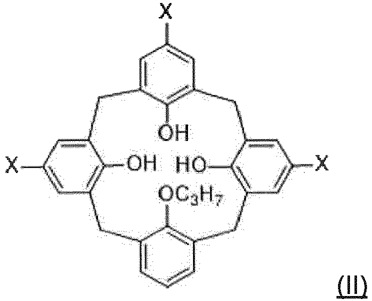 dans lequel :
- X est un groupement -(CH2)-CO2Y, et
- Y est un métal alcalin choisi parmi les sels de sodium, de potassium, d'ammonium, amino (organique) ou magnésium,
ou l'un de ses sels pharmaceutiquement acceptables,
pour la préparation d'un vaccin contre le virus de la grippe ou d'un antigène vaccinal du virus de la grippe par fragmentation d'une membrane biologique associée audit antigène vaccinal par mise en contact de ladite membrane biologique avec au moins ledit calixarène, ladite étape de fragmentation étant réalisée à une température allant de 4 à 25°C, et à une concentration dudit calixarène de 1%.

7. Vaccin selon la revendication 4 ou la revendication 5, pour son utilisation comme médicament dans le traitement ou la prévention de la grippe.

## Patentansprüche

1. Verfahren zur Herstellung eines fragmentierten Impfstoffantigens des Grippevirus, umfassend einen Schritt der Fragmentierung einer mit dem Impfstoffantigen assoziierten biologischen Membran durch Inkontaktbringen der biologischen Membran mit mindestens einem Calixaren der Formel (II): 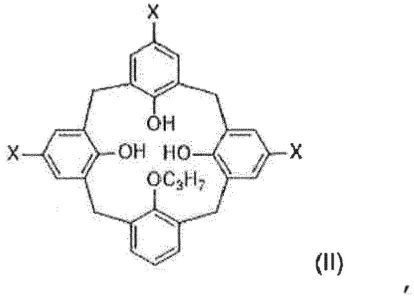 worin:
- X eine Gruppe -(CH₂)-CO₂Y- ist und
- Y ein Alkalimetall, ausgewählt aus den Salzen von Natrium, Kalium, Ammonium, (organischem) Amin oder Magnesium, ist,
oder einem seiner pharmazeutisch annehmbaren Salze,
wobei der Fragmentierungsschritt bei einer Temperatur von 4 bis 25°C und bei einer Konzentration des Calixarens von 1% durchgeführt wird.

2. Verfahren nach Anspruch 1, wobei das Grippevirus aus Influenzavirus A und Influenzavirus B ausgewählt ist.

3. Verfahren nach einem der vorhergehenden Ansprüche, das außerdem einen Schritt der Dialyse des Impfstoffantigens umfasst.

4. Impfstoff gegen das Grippevirus, der mittels Durchführung des Verfahrens nach einem der Ansprüche 1 bis 3 hergestellt werden kann, umfassend ein Calixaren der Formel (II) in Exzipientenform in einer Menge von 0,1 bis 1000 pg, bezogen auf das Gesamtgewicht des Impfstoffs.

5. Impfstoff nach Anspruch 4, wobei das Impfstoffantigen ein Membranprotein viralen Ursprungs umfasst.

6. Verwendung eines Calixarens der Formel (II): 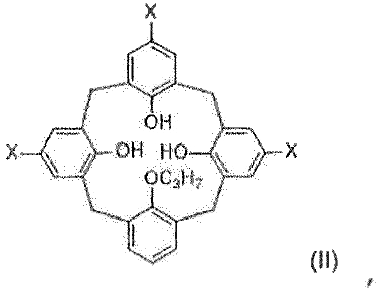 worin:
- X eine Gruppe -(CH₂)-CO₂Y- ist und
- Y ein Alkalimetall, ausgewählt aus den Salzen von Natrium, Kalium, Ammonium, (organischem) Amin oder Magnesium, ist,
oder eines seiner pharmazeutisch annehmbaren Salze,
zur Herstellung eines Impfstoffs gegen das Grippevirus oder eines Impfstoffantigens des Grippevirus durch Fragmentierung einer mit dem Impfstoffantigen assoziierten biologischen Membran durch Inkontaktbringen der biologischen Membran mit mindestens dem Calixaren, wobei der Fragmentierungsschritt bei einer Temperatur von 4 bis 25°C und bei einer Konzentration des Calixarens von 1% durchgeführt wird.

7. Impfstoff nach Anspruch 4 oder Anspruch 5 zur Verwendung als Medikament bei der Behandlung oder Vorbeugung der Grippe.

## Claims

1. A process for preparing a fragmented vaccine antigen of flu virus, comprising a step of fragmenting a biological membrane associated with said vaccine antigen by bringing said biological membrane into contact with at least one calixarene of formula (II): 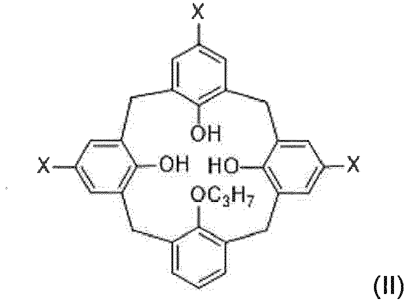 in which :
- X is a -(CH₂)-CO₂Y group, and
- Y is an alkali metal chosen among sodium, potassium, ammonium, amino (organic) or magnesium salts,
or a pharmaceutically acceptable salt thereof,
wherein said fragmenting step is performed at a temperature ranging from 4 to 25°C, and at a concentration of said calixarene of 1%.

2. The process according to claim 1, wherein said flu virus is chosen from influenza viruses A and influenza viruses B.

3. The process according to anyone of the preceding claims, also comprising a step of dialyzing of said vaccine antigen.

4. A vaccine against flu virus that can be produced by carrying out the process as defined in any one of claims 1 to 3, comprising a calixarene of formula (II) in excipient format in an amount of from 0.1 to 1000 µg in the total weight of the vaccine

5. The vaccine according to claim 4, wherein said vaccine antigen comprises a membrane protein of viral origin.

6. Use of a calixarene of formula (II): 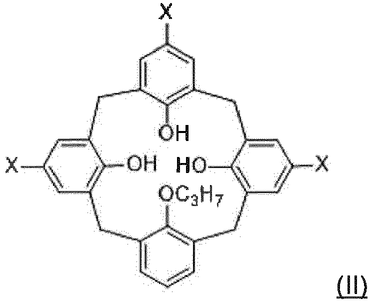 in which :
- X is a -(CH₂)-CO₂Y group, and
- Y is an alkali metal chosen among sodium, potassium, ammonium, amino (organic) or magnesium salts,
or a pharmaceutically acceptable salt thereof,
for the preparation of a vaccine against flu virus or of a vaccine antigen of flu virus by fragmenting a biological membrane associated with said vaccine antigen by bringing said biological membrane into contact with the at least one calixarene, said fragmenting step being performed at a temperature ranging from 4 to 25°C, and at a concentration of said calixarene of 1%.

7. Vaccine according to claim 4 or 5, for its use as a medicament for treating or preventing the flu.
